# EUROPÄISCHE PATENTSCHRIFT

(11) **EP 3 735 996 B1**
(45) Veröffentlichungstag und Bekanntmachung des Hinweises auf die Patenterteilung: **27.09.2023**
(21) Anmeldenummer: 19172988.8
(22) Anmeldetag: 07.05.2019
(51) Int. Cl.: A61M 60/00, A61M 1/36, A61M 25/00

(54) **BIDIREKTIONALE PERFUSIONSKANÜLE**
BI-DIRECTIONAL PERFUSION CANNULA
CANULE DE PERFUSION BIDIRECTIONNELLE

(43) Veröffentlichungstag der Anmeldung: 11.11.2020
(73) Patentinhaber: Free Life Medical GmbH, 52080 Aachen (DE)
(72) Erfinder: Hildinger, Karl-Heinz, 52080 Aachen (DE); Schmitz, Bernhard, 53937 Schleiden (DE); Autschbach, Rüdiger, 52074 Aachen (DE)
(74) Vertreter: Gille Hrabal Partnerschaftsgesellschaft mbB Patentanwälte

(56) Entgegenhaltungen:
- WO-A1-2019/075020
- US-A1- 2005 085 769
- US-A1- 2018 043 085

## Beschreibung

Die Erfindung betrifft eine medizinische Vorrichtung zur bidirektionalen Perfusion mit sauerstoffangereichertem Blut, insbesondere eine ECMO-Kanüle. Die Erfindung betrifft ferner ein Perfusionssystem mit der medizinischen Vorrichtung und einem Oxygenator, insbesondere einem ECMO-Oxygenator.

Um den Organismus beispielsweise während eines chirurgischen Eingriffs am Herzen weiterhin mit sauerstoffhaltigem Blut zu versorgen, wird in einigen Fällen sauerstoffangereichertes Blut in eine Arterie in Richtung des Herzens gepumpt, also in proximale Richtung. Ein Körperbereich in entgegengesetzter Richtung, also in distaler Richtung, kann dadurch Sauerstoffmangel erleiden. Daher gibt es medizinische Perfusionsvorrichtungen z.B. zur Extrakorporalen Membranoxygenierung (ECMO), die über eine zusätzlich Leitung einen distalen Bereich des Patienten mit sauerstoffangereichertem Blut versorgen. Um diese zusätzliche Leitung einzusparen, gibt es Systeme wie z.B. in der Druckschrift EP2694148A1 oder EP0619745A1 beschrieben, die ein Seitenloch in einem Abschnitt der medizinischen Perfusionsvorrichtung vorsehen, der genau innerhalb des Blutgefäßes und mit dem Seitenloch in distale Richtung zeigend zu positionieren ist. Um einen verbessert gerichteten Rückfluss zu erhalten, werden in den Druckschriften US2018043085A und WO 2019/075020 A1 kappenförmige Umlenkabdeckungen vorgeschlagen. Aufgrund der Beschädigungsgefahr des Blutgefäßes vor allem beim Entfernen der Vorrichtung zur bidirektionalen Perfusion kann die Umlenkabdeckung nur in einem gewissen Maße radial an dem Seitenloch hervorstehen, was wiederum einen Einfluss auf die Menge des möglichen Rückflusses haben kann. Die Druckschrift US2005085769A1 offenbart radial aufklappbare, konisch geformte Klappen über Seitenöffnungen.

Es ist Aufgabe der Erfindung, eine weiterentwickelte bidirektionale Perfusionsvorrichtung bereitzustellen, insbesondere zum Erreichen einer besonders hohe Rückflussmenge ohne ein überhöhtes Risiko einer Gefäßbeschädigung.

Zur Lösung der Aufgabe dient eine Vorrichtung gemäß Anspruch 1. Vorteilhafte Ausführungsformen ergeben sich aus den Unteransprüchen.

Zur Lösung der Aufgabe dient eine medizinische Vorrichtung zur bidirektionalen Perfusion mit sauerstoffangereichertem Blut, insbesondere eine ECMO-Kanüle. Die Vorrichtung umfasst einen Schlauchkörper und ist so eingerichtet, dass der Schlauchkörper in ein Blutgefäß eingeführt werden kann. Der Schlauchkörper erstreckt sich von einem ersten Ende zu einem zweiten Ende, um sauerstoffangereichertes Blut von einem Einlass an dem ersten Ende in eine Vorwärtsrichtung zu einem Auslass an dem zweiten Ende fließen zu lassen und aus dem Auslass in Vorwärtsrichtung abzugeben. Zwischen dem Einlass und dem Auslass ist mindestens ein Seitenloch mit einem Umlenkkörper vorhanden. Der Umlenkkörper befindet sich außen am Schlauchkörper über dem Seitenloch, um aus dem Seitenloch austretendes sauerstoffangereichertes Blut in eine entgegen der Vorwärtsrichtung gerichteten Rückwärtsrichtung umzulenken.

Der Umlenkkörper umgibt den Schlauchkörper (engl. "surround"). Eine besonders große Rückflussmenge zur Perfusion kann so ermöglicht werden, ohne ein überhöhtes Risiko einer Gefäßbeschädigung beim Entfernen des Schlauchkörpers zu erhalten.

Perfusion meint ein Durchströmen oder Durchbluten eines Gefäßes wie z.B. einer Arterie. Sauerstoffangereichertes Blut ist Blut, dessen Sauerstoffgehalt erhöht wurde, z.B. durch einen Oxygenator, insbesondere ein ECMO-Oxygenator, worauf unten noch genauer eingegangen werden wird. Eine ECMO-Kanüle ist eine flexible Kanüle, die gebogen werden kann, um in ein Gefäß, insbesondere eine Femoralarterie, eingebracht zu werden. Grundsätzlich kann es sich bei der medizinischen Vorrichtung um eine blutführende Vorrichtung im Bereich der Herz-, Gefäß- und Thorax-Chirurgie handeln, bei der sich der Einlass des Schlauchkörpers außerhalb eines Patientenkörpers und der Auslass innerhalb des Blutgefäßes befinden. Insbesondere kann die Vorrichtung eine ECLS-Kanüle und/oder der Oxygenator ein ECLS-Oxygenator sein. Wenn die Vorrichtung mit dem Schlauchkörper in ein Blutgefäß eingeführt wird, erfolgt dies durch eine Öffnung, die in eine Gefäßwand des Blutgefäßes eingebracht wurde, was auch Punktieren genannt wird. Insbesondere kann ein Dilatator oder ein anderes Punktierwerkzeug mit einer Spitze durch den Schlauchkörper geführt werden, um die Öffnung in die Gefäßwand einzubringen. Allgemein wird der Dilatator bzw. das Punktierwerkzeug nach einer Punktion aus dem Schlauchkörper entfernt, so dass der Schlauchkörper innen frei zum Leiten eines Durchflusses bereitsteht. Das Einführen der medizinischen Vorrichtung zur bidirektionalen Perfusion in ein Blutgefäß kann auch als Kanülieren bezeichnet werden.

In einer Ausgestaltung überdeckt der Umlenkkörper ein Seitenloch oder mehrere über den Umfang beabstandete Seitenlöcher vollständig. Grundsätzlich ist auch ein lediglich teilweises Überdecken des mindestens einen Seitenloches möglich. Das Seitenloch sollte zumindest überwiegend durch den Umlenkkörper überdeckt werden. Vorzugsweise ist die Längserstreckung eines Abschnitts des Umlenkkörpers, der nicht mit dem Schlauchkörper unmittelbar verbunden oder befestigt ist, mindestens so groß wie der Durchmesser des Seitenloches und/oder höchstens fünf Mal so groß wie der Durchmesser des Seitenloches. Vorzugsweise ist das Seitenloch in Längsrichtung mittig oder in einer oberen Hälft des Abschnitts des Umlenkkörpers angeordnet, der nicht mit dem Schlauchkörper unmittelbar verbunden oder befestigt ist. Insbesondere erstreckt sich der Umlenkkörper ausgehend von der Mantelfläche des Schlauchkörpers in Rückwärtsrichtung über das mindestens eine Seitenloch, so dass ein aus dem Seitenloch austretender Flüssigkeitsstrom durch den Umlenkkörper in Rückwärtsrichtung umgelenkt werden kann. Der Umlenkkörper erstreckt sich von einem oberen Rand des Seitenloches oder beabstandet vom oberen Rand des Seitenloches von der Mantelfläche zu einem freien Ende, das von der Mantelfläche beabstandet ist und/oder lose auf der Mantelfläche aufliegen kann, so dass ein Durchgang von dem Seitenloch zwischen dem Umlenkkörper und der Mantelfläche in die Umgebung vorhanden ist.

In einer Ausgestaltung ist der Umlenkkörper ein von dem Schlauchkörper separates Element und/oder der Umlenkkörper ist an dem Schlauchkörper befestigt oder mit dem Schlauchkörper verbunden. Eine Befestigung oder eine Verbindung von dem Umlenkkörper mit dem Schlauchkörper erfolgt oberhalb eines Seitenloches, d.h. in Vorwärtsrichtung, z.B. am oberen Rand des Seitenloches oder beabstandet vom oberen Rand des Seitenloches. Vorzugsweise erfolgt die Befestigung oder die Verbindung in einer geschlossenen Linie oder Fläche um den Schlauchkörper herum. In einer Ausgestaltung erfolgt das Befestigen oder Verbinden des Umlenkkörpers am bzw. mit dem Schlauchkörper durch eine stoffschlüssige Verbindung, eine Klebverbindung, eine Fügeverbindung und/oder eine Kaltverschweißung, insbesondere eine Kunststoffkaltverschweißung. Vorzugsweise wird die Mantelfläche des Schlauchkörpers zunächst mit einem Lösungsmittel behandelt und/oder angelöst, d.h., nur innerhalb einer äußeren Oberflächenschicht gelöst oder teilweise gelöst. Insbesondere erfolgt erst anschließend das Befestigen oder stoffschlüssig Verbinden, insbesondere Kleben. Bevorzugt wird Tetrahydrofuran (THP) eingesetzt. Grundsätzlich ist es auch möglich, den Umlenkkörper einstückig mit dem Schlauchkörper auszuführen. Die oben beschriebenen alternativen oder ergänzenden Arten der Verbindung oder Befestigung können auch im Folgenden angewendet werden, wenn ein Verbinden oder Befestigen beschrieben wird. Bevorzugt ist ein befestigter Bereich flächig oder vollflächig befestigt.

In einer Ausführungsform ist der Umlenkkörper konusartig, insbesondere stumpfkegelförmig. Eine besonders große Rückflussmenge zur Perfusion kann so erhalten werden, ohne ein überhöhtes Risiko einer Gefäßbeschädigung beim Entfernen des Schlauchkörpers zu erhalten. Ein konusartiger Umlenkkörper ermöglicht eine besonders große rückwärtige Fließquerschnittsfläche. Beim Entfernern kann die punktierte Öffnung im Blutgefäß über den gesamten Umfang näherungsweise gleichförmig aufgeweitet und ein Beschädigungsrisiko durch einen lokal begrenzten Vorsprung, wie dies beispielsweise bei einer radial hervorstehenden Kappenabdeckung der Fall wäre, vermieden werden. Die punktierte Öffnung ist grundsätzlich dehnbar, jedoch im gespannten Zustand anfällig für ein Einreißen, wobei sich eine örtlich begrenzte Beschädigungsstelle am Rand der Öffnung sich leicht ausweiten und eine größere Gefäßbeschädigung verursachen kann. Ein konusartiger Umlenkkörper hat eine schräge Kontur bezogen auf eine Mittelachse des Schlauchkörpers. Insbesondere ist der Umlenkkörper konusförmig und/oder erstreckt sich konusartig um den Schlauchkörper. Vorzugsweise steht der Umlenkkörper über den gesamten Umfang konusartig von der Mantelfläche des Schlauchkörpers ab. Insbesondere bildet der Umlenkkörper eine schirmartige Form oder eine Schirmform wie ein Regenschirm. In einer Ausgestaltung bildet der Schlauchkörper einen Vorsprung, der sich in Rückwärtsrichtung über den Schlauchkörper erstreckt. Der Vorsprung umgibt den Schlauchkörper in Umfangrichtung.

In einer Ausführungsform ist der Umlenkkörper trichterartig, wobei ein zylindrischer Abschnitt des trichterartigen Umlenkkörpers mit dem Schlauchkörper verbunden ist und ein konischer Abschnitt des trichterartigen Umlenkkörpers von dem Schlauchkörper absteht. Ein besonders großer Rückfluss ohne ein überhöhtes Gefäßbeschädigungsrisiko kann so erzielt werden. Der konische Abschnitt, d.h. ein konusartiger oder konusförmiger Abschnitt, hat ein freies, umlaufendes Ende und ein fixiertes, umlaufendes Ende am Übergang zum zylindrischen Abschnitt. Der zylindrische Abschnitt, d.h. ein zylinderartiger oder zylinderförmiger Abschnitt, ist insbesondere über den gesamten Umfang fest mit dem Schlauchkörper verbunden, vorzugsweise wasserdicht und/oder stoffschlüssig. In einer Ausgestaltung ist der Umlenkkörper so beschaffen oder so am Schlauchkörper befestigt, dass der konische Abschnitt des Umlenkkörpers seitlich verschwenkt werden kann. In einer Ausgestaltung ist der Umlenkkörper so beschaffen, dass sich der konische Abschnitt des Umlenkkörpers durch eine Druckeinwirkung verformen lässt, beispielsweise durch einen austretenden Flüssigkeitsstrom aus dem Seitenloch oder bei einem Drücken gegen einen Rand der Öffnung in einem Blutgefäß beim Entfernen der Vorrichtung.

In einer Ausführungsform ist der Umlenkkörper ein am Schlauchkörper befestigter Streifen oder der Umlenkkörper umfasst einen oder mehrere Streifen, bevorzugt genau einen Streifen. Ein Streifen ist ein flaches Materialstück, das länger ist als breit, vorzugsweise rechteckförmig. Insbesondere ist die Breite des Streifens mindestens so groß wie der Durchmesser des Schlauchkörpers und/oder höchstens fünf Mal so groß. Insbesondere ist der Streifen so am Schlauchkörper befestigt, dass der Streifen nicht zerstörungsfrei lösbar ist. Die Dicke des Streifens erstreckt sich radial. Insbesondere hat der Streifen eine einheitliche Dicke. Auf die Dicke wird unten im Zusammenhang mit der Dicke des Umlenkkörpers noch genauer eingegangen. Der Streifen kann einen Abschnitt zum flächigen Befestigen an der Mantelfläche des Schlauchkörpers und einen davon abgewinkelten Abschnitt zum Ausbilden eines freien Endes aufweisen. Der Streifen kann teilweise an der Mantelfläche des Schlauchkörpers oberhalb eines Seitenloches, d.h. in Vorwärtsrichtung, befestigt werden und der nicht befestigte Abschnitt des Streifens kann verformt werden, um von der Mantelfläche abzustehen und sich über das Seitenloch in Rückwärtsrichtung zu erstrecken. In einer Ausgestaltung sind mehrere Streifen überlappend am Schlauchkörper befestigt. In einer Ausgestaltung wird der Streifen in Umfangrichtung oder zur Umfangrichtung geneigt um den Schlauchkörper gewickelt. Die Wickelrichtung ist dabei parallel zur Längsrichtung des Streifens oder zumindest im Wesentlichen in Richtung zur längsten Erstreckung des Streifens. Die Umfangrichtung liegt auf einer Querschnittsebene zur Mittelachse des Schlauchkörpers. Insbesondere wird der Streifen mit mindestens einer Wicklung und/oder höchstens fünf Wicklungen, jeweils 360° pro Wicklung, um den Schlauchkörper gewickelt. In einer Ausgestaltung ist der Umlenkkörper oder die Wicklung des Streifens spiralartig, spiralförmig, helixartig oder helixförmig um den Schlauchkörper. Ein besonders großer Rückfluss kann so ermöglicht und die Vorrichtung durch eine Drehbewegung besonders leicht aus einem Blutgefäß entfernt werden. In einer Ausgestaltung ist der Streifen dreieckartig oder dreieckförmig. In einer Ausgestaltung umgibt der Streifen den Schlauchkörper schleifenartig (engl. ribbon-like) oder schleifenförmig. Vorzugsweise verläuft der Streifen V-artig. Insbesondere zeigt eine Spitze einer V-Form oder einer dreieckartigen Form des Streifens in die Vorwärtsrichtung und/oder ist mit dem Schlauchkörper oberhalb von dem Seitenloch befestigt, so dass der rückwärtsliegende Teil des Streifens das Seitenloch überdeckt. Der rückwärtig liegende Teil kann den Schlauchkörper beidseitig umgreifen und/oder an einer Seite des Schlauchkörpers gegenüber von dem überdeckten Seitenloch insbesondere vollständig vollflächig an dem Schlauchkörper befestigt sein. Insbesondere treffen zwei gegenüberliegende Enden eines Streifens X-förmig durch ein übereinander kreuzten aufeinander und/oder laufen V-artig unter einem stumpfen Winkel aufeinander zu. Vorzugsweise wird der Streifen nur mit einer Längskante an der Mantelfläche des Schlauchkörpers befestigt, um beispielsweise einen konusförmigen oder trichterförmigen Umlenkkörper zu bilden.

In einer Ausführungsform ist der Umlenkkörper ballonartig. Eine besonders große Rückflussmenge zur Perfusion kann so erhalten werden, ohne ein überhöhtes Risiko einer Gefäßbeschädigung beim Entfernen des Schlauchkörpers zu erhalten. Insbesondere ist der ballonartige Umlenkkörper in einer geschlossenen und/oder wasserdichten Art und Weise um das mindestens eine Seitenloch mit der Mantelfläche verbunden oder daran befestigt, so dass ein Flüssigkeitsstrom aus dem Seitenloch ins Innere des ballonartigen Umlenkkörpers strömt und diesen ausfüllen kann. Bevorzugt ist der ballonartige Umlenkkörper dünnwandig und/oder faltbar. Die Vorrichtung kann dadurch mit dem ballonartigen Umlenkkörper in einem entleerten Zustand, bei dem der Umlenkkörper radial kaum übersteht, in ein Blutgefäß eingeführt und/oder entfernt werden. Im Betrieb kann der ballonartige Umlenkkörper durch den Flüssigkeitsstrom aus dem Seitenloch gefüllt werden und im gefüllten Zustand weit radial hervorsteht. Bevorzugt erstreckt sich ein ballonartiger Umlenkkörper ringförmig um den Schlauchkörper herum. Ein ballonartiger Umlenkkörper umfasst eine oder mehrere Austrittsöffnungen in Rückwärtsrichtung oder begrenzt einen Durchgang zusammen mit der Mantelfläche in Rückwärtsrichtung von dem mindestens einen Seitenloch.

In einer Ausführungsform ist der Umlenkkörper so eingerichtet, dass sich der Umlenkkörper durch eine Druckeinwirkung eines aus dem Seitenloch austretenden Flüssigkeitsstroms bewegen oder verformen lässt, insbesondere verdrängen oder elastisch expandieren lässt. Eine besonders große Rückflussmenge zur Perfusion kann so erhalten werden, ohne ein überhöhtes Risiko einer Gefäßbeschädigung beim Entfernen des Schlauchkörpers zu erzielen. Das Bewegen, Verformen, Verdrängen oder elastische Expandieren findet allgemein nur so lange statt, bis ein mechanisches Gleichgewicht zwischen der Druckeinwirkung und dem Umlenkkörper erreicht ist. Nachdem beispielsweise der Schlauchkörper mit dem Umlenkkörper in dem Blutgefäß platziert ist, wird in weniger als einer Sekunde das mechanische Gleichgewicht erreicht sein, wenn zu Beginn des Betriebs ein Flüssigkeitsstrom aus dem Seitenloch austritt und den Umlenkkörper entsprechend bewegt, verformt, verdrängt oder elastisch expandiert wird.

In einer Ausführungsform ist die medizinische Vorrichtung so eingerichtet, dass je größer eine Durchflussmenge des aus dem Seitenloch austretenden Flüssigkeitsstroms ist, desto größer wird eine rückwärtige Flussquerschnittsfläche, die einen Rückfluss in die Rückwärtsrichtung begrenzt. Je größer der Durchfluss durch den Schlauchkörper ist, desto größer ist der Durchfluss durch das Seitenloch und damit die Bewegung oder Verformung des Umlenkkörpers. Die rückwärtige Flussquerschnittsfläche und damit die Rückflussmenge steigen dann mit größer werdendem Durchfluss in Vorwärtsrichtung an. Wenn mehrere Seitenlöcher vorgesehen sind, die gemeinsam von einem einzigen sich in Umfangrichtung erstreckenden Umlenkkörper überdeckt werden, kann der Umlenkkörper in den jeweiligen Bereichen in Umfangrichtung über den Seitenlöchern radial ausgelenkt werden. Ein freies Ende des Umlenkkörpers verläuft dann wellenförmig um den Schlauchkörper herum. Alternativ oder ergänzend kann die Umfanglänge, d.h., die geschlossene Bogenlänge, des freien Endes des Umlenkkörpers auf diese Weise insgesamt elastisch expandiert werden. Die rückwärtige Flussquerschnittsfläche kann so vergrößert, der Rückfluss gesteigert und die Vorrichtung besonders leicht entfernt werden. Eine Druckeinwirkung von einem aus dem Seitenloch austretenden Flüssigkeitsstroms ist allgemein geringer als eine Druckeinwirkung, die beim Entfernen der Vorrichtung entsteht. In Folge der beim Entfernen entstehenden Druckeinwirkung durch das Gewebe und insbesondere den Rand der punktierten Öffnung im Blutgefäß auf den Umlenkkörper kann ein geringes Beschädigungsrisiko für ein Blutgefäß durch die Vorrichtung gemäß dieser Ausführungsform erhalten werden. Der Umlenkkörper kann sich hinsichtlich seiner radialen Ausdehnung und Form an die Größe und Kontur der Öffnung in dem Blutgefäß beim Entfernen anpassen. Insbesondere ist die medizinische Vorrichtung zur bidirektionalen Perfusion so eingerichtet, dass eine Durchflussmenge in Vorwärtsrichtung zwischen 1 l/min (Liter pro Minute) und 10 l/min liegen kann. Insbesondere beträgt eine Durchflussmenge aus einem Seitenloch mindestens 0,01 l/min. Vorzugsweise beträgt ein Druck durch ein aus dem Seitenloch austretenden Flüssigkeitsstrom im Betrieb mindestens ungefähr zwei zehntel oder drei zehntel bar. Insbesondere ist der Umlenkkörper so eingerichtet, dass sich der Umlenkkörper durch eine Druckdifferenz von mindestens 0,01 bar relativ zum Schlauchkörper bewegen oder verformen lässt. Die Druckdifferenz kann z.B. durch ein aus dem Seitenloch austretenden Flüssigkeitsstrom oder einem Kontakt mit einem Rand einer punktierten Öffnung in einem Blutgefäß resultieren.

In einer Ausgestaltung hat der Umlenkkörper eine Dicke von mindestens 1,1 mm und/oder höchstens 2 mm. Durch ein Verschwenken, Auslenken und Verlagern des Umlenkkörpers relativ zur Mantelfläche kann so der Rückfluss vergrößert und das Entfernen erleichtert werden. In einer Ausgestaltung hat der Umlenkkörper eine Dicke von mindestens 0,6 mm und/oder höchstens 1 mm. Zusätzlich zum Verschwenken, Auslenken und Verlagern kann der Umlenkkörper lokal gewölbt werden und so der Rückfluss vergrößert und das Entfernen erleichtert werden.

In einer bevorzugten Ausführungsform hat der Umlenkkörper eine Dicke von mindestens 0,05 mm und/oder höchstens 0,5 mm. Im Betrieb kann eine besonders große rückwärtige Flussdurchschnittsfläche erzielt werden. Grundsätzlich kann darüber hinaus die Vorrichtung ganz besonders schonend aus einem Blutgefäß entfernt werden, wenn der Umlenkkörper beim Auftreffen auf einen Rand der Öffnung des Blutgefäßes umgeschlagen oder umgestülpt wird, d.h. die im Betrieb der Mantelfläche des Schlauchkörpers zugewandte Oberfläche des Umlenkkörpers nach außen umgeklappt wird, was durch eine Dicke von 0,08 bis 0,1 mm weiter begünstigt wird.

In einer Ausführungsform umfasst der Umlenkkörper ein Material oder ist der Umlenkkörper aus einem Material hergestellt, das für ein elastisches Verformen oder ein elastisches Expandieren im Betrieb vorgesehen ist, insbesondere zum Vergrößern der rückwärtigen Flussquerschnittsfläche in Abhängigkeit von einer Durchflussmenge aus einem aus dem Seitenloch austretenden Flüssigkeitsstrom aus sauerstoffangereichertem Blut. In einer Ausführungsform enthält der Umlenkkörper ein Elastomer oder besteht daraus. In einer Ausführungsform enthält der Umlenkkörper Polyurethan (PU) oder ist daraus hergestellt. Alle diese Ausführungsformen in Bezug auf die Beschaffenheit des Materials des Umlenkkörpers ermöglichen eine besonders große Rückflussmenge zur Perfusion, ohne ein überhöhtes Risiko einer Gefäßbeschädigung beim Entfernen des Schlauchkörpers zu erhalten.

In einer Ausführungsform werden mindestens zwei und/oder höchstens zehn Seitenlöcher von einem einzigen Umlenkkörper überdeckt. Eine besonders große Rückflussmenge zur Perfusion kann so erzielt werden, ohne ein überhöhtes Risiko einer Gefäßbeschädigung beim Entfernen des Schlauchkörpers zu erhalten. Insbesondere befinden sich die Seitenlöcher alle auf einer gleichen Höhe entlang der Mittelachse des Schlauchkörpers und/oder sind gleichmäßig in Umfangrichtung verteilt. Vorzugsweise haben die Seitenlöcher alle einen gleichen Durchmesser.

In einer Ausgestaltung ist das Seitenloch oder eines der Seitenlöcher in einem zentralen Bereich über die Mittelachse des Schlauchkörpers betrachtet angeordnet. In einer Ausgestaltung ist das Seitenloch oder eines der Seitenlöcher in einem oberen Bereich angeordnet, das dem zweiten Ende zugewandt ist. Der zentrale Bereich und/oder der obere Bereich haben eine Längserstreckung, also parallel zur Mittelachse, von ungefähr einem Drittel oder Viertel der Länge des Schlauchkörpers.

In einer Ausführungsform sind mindestens zwei Umlenkkörper vorhanden, die in der Vorwärtsrichtung voneinander beabstandet sind und sich jeweils über mindestens einem Seitenloch befinden. Eine besonders große Rückflussmenge zur Perfusion kann so erhalten werden, ohne ein überhöhtes Risiko einer Gefäßbeschädigung zu erhalten.

In einer Ausführungsform ist eine Vielzahl von Stellen in einer Wandung des Schlauchkörpers zum Einbringen eines Seitenloches vorgesehen und/oder nur eine oder mehrere Stellen davon sind zum Erhalt eines Seitenloches gelocht. Eine besonders große Rückflussmenge zur Perfusion kann so erhalten werden, ohne ein überhöhtes Risiko einer Gefäßbeschädigung zu erhalten. Insbesondere erfolgt die Auswahl der Stellen in Abhängigkeit von einer gewünschten Rückflussmenge in Rückwärtsrichtung relativ zur Durchflussmenge aus dem Auslass in Vorwärtsrichtung. Die Auswahl kann auch die Rückfluss-Charakteristik beeinflussen. Vorzugsweise ist ein gelochtes Metallblech vorgesehen, das mit einen Kunststoff umspritzt ist. Ein Lochen kann dann durch ein Entfernen des Kunststoffs an den Stellen der Löcher im Metallblech erfolgen, beispielsweise durch ein Durchstechen.

In einer Ausführungsform ist ein Druckmessschlauch vorgesehen, der in Vorwärtsrichtung zum Seitenloch führt, damit ein Flüssigkeitsdruck des aus dem Seitenloch austretenden Flüssigkeitsstroms gemessen werden kann. Eine besonders große Rückflussmenge zur Perfusion kann so sichergestellt werden, ohne ein überhöhtes Risiko einer Gefäßbeschädigung zu erhalten. Beispielsweise kann bei einem gemessenen, geringem Flüssigkeitsdruck die Lage des Schlauchkörpers im Blutgefäß verändert oder der Durchfluss am Einlass erhöht werden. Insbesondere ist der Druckmessschlauch auf der Mantelfläche des Schlauchkörpers befestigt, verläuft parallel zum Schlauchkörper und/oder ist auf der Mantelfläche mit einer dünnen Kunststoffschicht überspritzt. Der Durchmesser des Druckmessschlauchs entspricht ungefähr dem Durchmesser des Seitenloches. Vorzugsweise grenzt der Druckmessschlauch ungefähr an einen nächstgelegenen Rand des Seitenloches an. Vorzugsweise verläuft der Druckmessschlauch zentriert zum Seitenloch.

In einer Ausführungsform weist der Schlauchkörper eine dreieckartige Querschnittskontur der äußeren Mantelfläche auf. Eine dreieckartige Querschnittskontur der äußeren Mantelfläche mit einem Umlenkkörper, der sich über dem Seitenloch befindet und den Schlauchkörper umgibt, ermöglicht eine besonders große Rückflussmenge zur Perfusion, ohne ein überhöhtes Risiko einer Gefäßbeschädigung zu erhalten. Vorzugsweise ist das Seitenloch in einer ebenen Fläche der dreieckartigen Querschnittskontur eingebracht. In einer Ausgestaltung ist alternativ eine polygonale Querschnittskontur mit mehr als drei Ecken, bevorzugt vier bis sechs Ecken, vorgesehen. Alternativ ist eine ovale Querschnittskontur vorgesehen.

Ein Aspekt der Erfindung betrifft ein Perfusionssystem mit der oben beschriebenen, erfindungsgemäßen medizinischen Vorrichtung zur bidirektionalen Perfusion und einem Oxygenator, insbesondere einem ECMO-Oxygenator. Der Oxygenator ist an den Einlass der medizinischen Vorrichtung zur bidirektionalen Perfusion angeschlossen, so dass sauerstoffangereichertes Blut in den Einlass gepumpt werden kann. Das Perfusionssystem umfasst ebenfalls eine Pumpe zum Pumpen des sauerstoffangereicherten Bluts in den Einlass. Die Pumpe kann in dem Oxygenator integriert sein.

ECMO ist die Abkürzung für Extrakorporale Membranoxygenierung. Die EMCO gewährleistet einen Gasaustausch, indem Blut von einem Patienten entnommen, durch einen Oxygenator mit Sauerstoff angereichert und anschließend wieder dem Patienten zugeführt wird, so dass das Patientenblut in dieser Weise zirkuliert wird. Der Gasaustausch zum Erhöhen des Sauerstoffgehalts des entnommenen Bluts findet insbesondere über eine semipermeable Membran statt, auf dessen einer Seite das entnommene Patientenblut fließt und auf der anderen Seite Sauerstoff eingeleitet wird.

Bidirektional bedeutet in zwei Richtungen. Beispielsweise wird bei einer bidirektionalen Vorrichtung in Form einer ECMO-Kanüle oder Katheter sauerstoffangereichertes Blut und/oder eine Flüssigkeit in die Vorwärtsrichtung in ein Blutgefäß geleitet, bevorzugt die Femoralarterie eines Beins. In einer Ausgestaltung ist die Vorwärtsrichtung proximal und/oder die Rückwärtsrichtung distal zum Patientenkörper orientiert. In proximaler Richtung kann sauerstoffangereichertes Blut zum Herzen befördert werden, während in distaler Richtung ein unterer Bereich eines Beins mit sauerstoffangereicherten Blut versorgt werden kann. Der Umlenkkörper erstreckt sich in Rückwärtsrichtung bis zu dessen Rand. Der Rand kann um mindestens ein Drittel oder die Hälfte des Umfangs des Schlauchkörpers durchgängig vom Schlauchkörper beabstandet sein oder darauf aufliegen, ohne daran befestigt zu sein. Der Rand kann teilweise oder daran angrenzend am Schlauchkörper befestigt sein. Der Rand kann ein freies Ende in Rückwärtsrichtung begrenzen. Die Ausdehnung des Umlenkkörpers in Umfangrichtung kann höchstens ein Drittel kleiner als ein Umfang des Schlauchkörpers sein.

Ein Schlauchkörper ist allgemein ein flexibler Hohlkörper, d.h. biegbar. In der Regel hat ein Schlauchkörper nur zwei gegenüberliegende Enden und/oder ist durchströmbar, d.h. der Schlauchkörper erlaubt ein Durchströmen vom ersten Ende in eine Vorwärtsrichtung entlang einer Mittelachse des Schlauchkörpers zum (gegenüberliegenden) zweiten Ende. Wird der Schlauchkörper gebogen, so erfolgt ein Durchfluss durch den Schlauchkörper entlang einer entsprechend gebogenen Mittelachse in Vorwärtsrichtung. Allgemein weisen ein Einlass am ersten Ende und/oder ein Auslass am zweiten Ende eine zu einer Mittelachse des Schlauchkörpers parallele oder koaxiale Durchgangsöffnung auf, die Kanülierung genannt wird. Der Schlauchkörper wird im Wesentlichen durch eine Wandung gebildet, die eine äußere Mantelfläche des Schlauchkörpers formt und sich vom ersten Ende bis zum zweiten Ende erstreckt. Die Mantelfläche ist biokompatible. In einer Ausgestaltung ist die Wandung mit einem Kunststoff, insbesondere einem Elastomer, bevorzugt Polyurethan (PU), hergestellt. In einer Ausgestaltung ist in der Wandung ein Stützdraht integriert. Bevorzugt ist der Stützdraht aus Metall und/oder von dem Kunststoff umspritzt. Vorzugsweise verläuft der Stützdraht spiralartig oder wendelartig um die Mittelachse des Schlauchkörpers. Insbesondere ist die Wandung mindestens 0,3 mm und/oder höchstens 2 mm dick. Bevorzugt ist die Wandung ungefähr 0,5 mm dick. Der Drahtdurchmesser des Stützdrahtes ist kleiner als die Wandungsdicke. Insbesondere ist eine Kunststoffschicht, die radial auf dem Stützdraht liegt, dünner als der Drahtdurchmesser. Die Vorrichtung ist insbesondere so eingerichtet, dass dessen Schlauchkörper bis zu dreißig Tage in einem Blutgefäß belassen werden kann. Insbesondere hat der Schlauchkörper einen im Wesentlichen konstanten Außenumfang vom ersten Ende bis zum zweiten Ende. Wenn der Schlauchkörper eine kreisförmige Querschnittskontur hat, liegt der Durchmesser des Schlauchkörpers bevorzugt zwischen 6 mm und 15 mm. Vorzugsweise ist das erste Ende mit einem Anschlussteil verbunden, insbesondere wasserdicht und/oder stoffschlüssig. Insbesondere ist das Anschlussteil so eingerichtet, dass ein Zuleitungsschlauch an das Anschlussteil angeschlossen werden kann. Beispielsweise führt der Zuleitungsschlauch zu einem Oxygenator. Ein Anschlussteil hat z.B. eine Länge von mindestens 20 % und/oder höchstens 90 % der Länge des Schlauchkörpers. Eine Länge wird entlang der Mittelachse gemessen. Bevorzugt ist das zweite Ende mit einem Kopfteil verbunden, insbesondere wasserdicht und/oder stoffschlüssig. Ein Kopfteil hat z.B. eine Länge von ungefähr 5 % bis 20 % der Länge des Schlauchkörpers. Bevorzugt haben das Kopfteil und/oder das Anschlussteil einen sich in die Vorwärtsrichtung verjüngenden Abschnitt und/oder unterscheidet sich hinsichtlich des Außenumfangs oder einer Oberflächenstruktur von dem Schlauchkörper.

Ein Seitenloch ist ein seitliches Loch, d.h. ein Durchgangsloch in der Wandung des Schlauchkörpers, insbesondere mit einem runden Querschnitt. Wenn die Wandung einen Stützdraht umfasst, erstreckt sich das Seitenloch zwischen zwei nebeneinander liegenden Stützdrahtabschnitten oder Drahtwindungen. Das Seitenloch erstreckt sich in einer Ausgestaltung im Wesentlichen radial zur Mittelachse des Schlauchkörpers und/oder ist zu einer radialen Geraden (radial zur Mittelachse) abgewinkelt, vorzugsweise unter einem spitzen Winkel zur radialen Geraden.

Ein Umlenkkörper, der sich außen am Schlauchkörper über dem Seitenloch befindet, ist dazu eingerichtet, einen radialen Abstand von einem Rand des Seitenloches an der Mantelfläche des Schlauchkörpers erzeugen zu können. Ein Teil des von dem Einlass zum Auslass des Schlauchkörpers strömenden Flusses von sauerstoffangereichertem Blut tritt aus dem Seitenloch aus und trifft im Wesentlichen radial auf den Umlenkkörper. Ein Umgeben bedeutet in Umfangrichtung umgeben. In einer Ausgestaltung bildet der Umlenkkörper gemeinsam mit der Mantelfläche einen Hohlraum, der bevorzugt einseitig in Vorwärtsrichtung geschlossen ist, so dass kein Fluss in Vorwärtsrichtung und/oder nur ein Rückfluss in Rückwärtsrichtung durch eine rückwärtige Fließquerschnittsfläche möglich sind.

Eine rückwärtige Fließquerschnittsfläche wird durch den Umlenkkörper und die Mantelfläche des Schlauchkörpers definiert. Die Fließquerschnittsfläche erstreckt sich auf einer Querschnittsebene zur Mittelachse des Schlauchkörpers. Die rückwärtige Fließquerschnittsfläche stellt die Engstelle (engl. "Bottleneck") für einen Rückfluss in die Rückwärtsrichtung dar. Eine Rückflussmenge des Rückflusses von sauerstoffangereichertem Blut aus dem mindestens einen Seitenloch, die durch den Umlenkkörper in die Rückwärtsrichtung umgelenkt wurde, wird somit von der rückwärtigen Fließquerschnittsfläche begrenzt. Je größer die rückwärtige Fließquerschnittsfläche ist, desto größer die mögliche Rückflussmenge. Eine Durchflussmenge und eine Rückflussmenge können z.B. in Liter pro Minute oder m³ pro Sekunde angegeben werden. Insbesondere ist der Umlenkkörper derart mit dem Schlauchkörper verbunden oder an dem Schlauchkörper befestigt, dass ein zerstörungsfreies Lösen des Umlenkkörpers vom Schlauchkörper nicht möglich ist. Insbesondere hat ein Umlenkkörper ein freies Ende in Rückwärtsrichtung, das über 360° von der äußeren Mantelfläche des Schlauchkörpers beabstandet sein und/oder lose darauf aufliegen kann.

Ein weiterer Aspekt der Offenbarung, der nicht beansprucht ist, betrifft eine Perfusionskanüle oder Katheter, wobei ein Schlauchkörper so eingerichtet ist, dass der Schlauchkörper in ein Blutgefäß eingeführt werden kann, wobei sich der Schlauchkörper von einem ersten Ende zu einem zweiten Ende erstreckt, um eine Flüssigkeit von einem Einlass an dem ersten Ende in eine Vorwärtsrichtung zu einem Auslass an dem zweiten Ende fließen zu lassen und aus dem Auslass in Vorwärtsrichtung abzugeben, wobei zwischen dem Einlass und dem Auslass mindestens ein Seitenloch mit einem Umlenkkörper vorhanden ist, der sich außen am Schlauchkörper über dem Seitenloch befindet, um einen aus dem Seitenloch austretenden Flüssigkeitsstrom in eine entgegen der Vorwärtsrichtung gerichteten Rückwärtsrichtung umzulenken, wobei der Umlenkkörper außen an dem Schlauchkörper befestigt ist, derart, dass sich der Umlenkkörper ausgehend von seiner Befestigung an dem Schlauchkörper mit einem freien Ende in Rückwärtsrichtung über das Seitenloch erstreckt, wobei das freie Ende von dem Schlauchkörper radial beabstandet ist oder lose außen auf dem Schlauchkörper aufliegt. Die oben im Zusammenhang mit der eingangs beschriebenen Erfindung beschriebenen Merkmale können mit diesem Aspekt der Erfindung kombiniert werden.

Ein weiterer Aspekt der Offenbarung, der nicht beansprucht ist, betrifft eine ECMO-Kanüle mit einer polygonalen, bevorzugt dreieckigen Querschnittskontur. Die oben im Zusammenhang mit der eingangs beschriebenen Erfindung beschriebenen Merkmale können mit diesem Aspekt der Erfindung kombiniert werden.

Nachfolgend werden weitere Ausführungsbeispiele der Erfindung auch anhand von Figuren näher erläutert.

Es zeigen:
- Figur 1:: Schematische Darstellung einer bidirektionalen Perfusionsvorrichtung, die mit dem Schlauchkörper in ein Blutgefäß eingeführt ist;
- Figur 2:: Detailansicht eines Schlauchkörpers mit einem umgebenden Umlenkkörper;
- Figur 3a:: Kreisförmige Querschnittskontur eines Schlauchkörpers der Fig. 2;
- Figur 3b:: Dreieckige Querschnittskontur eines Schlauchkörpers der Fig. 2;
- Figur 4: Schematische Darstellung einer bidirektionalen Perfusionsvorrichtung mit einem konischen Umlenkkörper;
- Figur 5: Darstellung einer bidirektionalen Perfusionsvorrichtung mit einem ballonartigem Umlenkkörper.

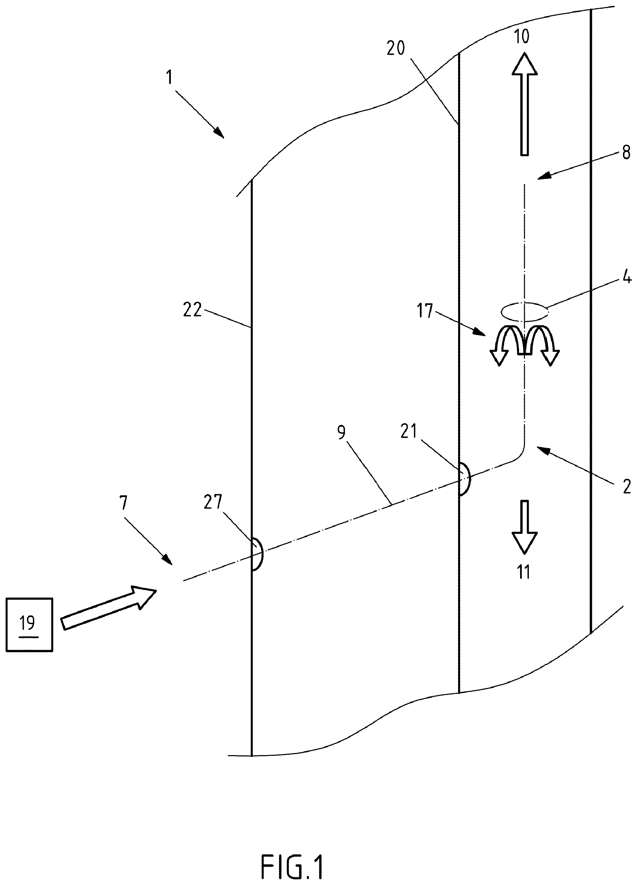

Die Figur 1 illustriert schematisch eine medizinische Vorrichtung zur bidirektionalen Perfusion, d.h., eine medizinische bidirektionale Perfusionsvorrichtung, die in Fig. 1 vereinfacht durch dessen Mittelachse 9 dargestellt ist. Minimalinvasiv wurde die Perfusionsvorrichtung mit dem Schlauchkörper 2 über eine kleine Inzision 27 durch die Patientenhaut 22 und das darunter liegende Gewebes bis zum Blutgefäß 20 geführt. Durch eine Punktion des Blutgefäßes 20 wurde eine Öffnung 21 in das Blutgefäß 20 eingebracht. An dem Schlauchkörper 2 ist ein Umlenkkörper 4 befestigt, der den Schlauchkörper 4 umgibt. Der Schlauchkörper 4 weist wie in Fig. 1 angedeutet eine geschlossene Linie um den Schlauchkörper 4 auf. Der Schlauchkörper 2 wurde mit dem Umlenkkörper 4 durch die Öffnung 21 in das Blutgefäß 20 eingeführt. Der Schlauchkörper 2 ist flexibel und passt sich innerhalb des Blutgefäßes 20 an den Verlauf des Blutgefäßes 20 an. Insbesondere ist die medizinische Vorrichtung der Figur 1 eine EMCO-Kanüle 1, durch die sauerstoffangereichertes Blut von einem ECMO-Oxygenator 19 in den Einlass 7 der EMCO-Kanüle 1 gepumpt wird. Das sauerstoffangereicherte Blut fließt hauptsächlich, insbesondere zu einem Anteil von mindestens 90%, in Vorwärtsrichtung 10 aus dem Auslass 8 proximal in die Femoralarterie. Ein kleiner Anteil davon tritt als ein Flüssigkeitsstrom 17 durch ein oder mehrere Seitenlöcher (in Fig. 1 nicht dargestellt) lateral aus und wird durch den Umlenkkörper 4 in die Rückwärtsrichtung 11 umgelenkt. Auf diese Weise wird auch der distale Bereich des Beins mit sauerstoffangereicherte Blut versorgt. Der Umlenkkörper 4 kann sich dabei an einer beliebigen Höhe, d.h., Position, entlang der Mittelachse 9 innerhalb des Blutgefäßes 20 befinden. Die EMCO-Kanüle 2 wird auf dem gleichen Weg aus dem Blutgefäß 20 entfernt, auf den sie zuvor eingeführt worden war.

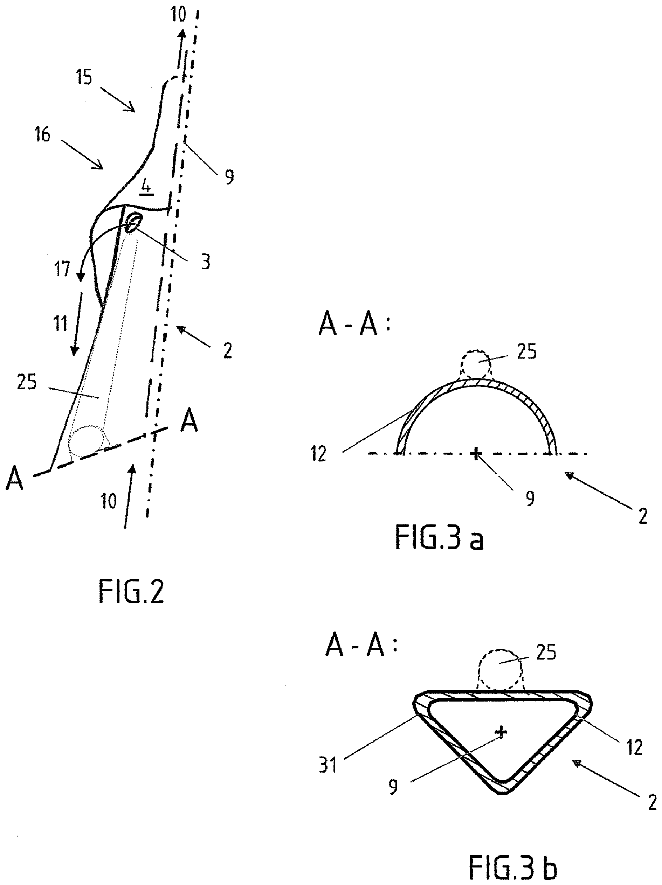

Die Figur 2 zeigt einen Ausschnitt von einem Schlauchkörper 2 einer bidirektionalen Perfusionsvorrichtung isometrisch in Vorwärtsrichtung 10, so dass das Seitenloch 3, über dem sich ein Umlenkkörper 4 befindet, zu sehen ist. Der Umlenkkörper 4 umgibt den Schlauchkörper 2. Ein insbesondere zylindrischer mit dem Schlauchkörper 2 verbundener Abschnitt 15 ist stoffschlüssig an einer Mantelfläche 26 des Schlauchkörpers 2 befestigt, bevorzugt geklebt. Ein insbesondere konischer von dem Schlauchkörper 2 abstehender Abschnitt 16 des Schlauchkörpers 2 erstreckt sich von dem Abschnitt 15 insbesondere trichterartig mit einem freien Ende 18 in die Rückwärtsrichtung 11. Der Umlenkkörper 4, der insbesondere aus einem dünnen Streifen aus einem biokompatiblen Elastomer besteht, wird durch den aus dem Seitenloch 3 austretenden Flüssigkeitsstrom 17 radial verdrängt und elastisch gedehnt. An den Stellen am Umfang des Schlauchkörpers 2, an denen keine Seitenlöcher 3 vorhanden sind und daher kein Flüssigkeitsstrom 17 austritt, liegt der Umlenkkörper 4 lose auf der Mantelfläche 26 auf und wird entsprechend gestrafft. Eine rückwärtige Flussquerschnittsfläche kann so vergrößert und die Rückflussmenge besonders mit zunehmender Durchflussmenge sehr effektiv gesteigert werden.

Wenn die Perfusionsvorrichtung aus einem Blutgefäß 20 entfernt wird, wie es anhand von Fig. 1 erläutert wurde, wird der Umlenkkörper 4 durch die Öffnung 21, die in das Blutgefäß 20 eingebracht wurde, zusammen mit dem Schlauchkörper 2 herausgezogen. Im Betrieb erstreckt sich der Umlenkkörper 4 über dem Seitenloch 3 in radialer Richtung weiter als eine Öffnung 21 in einem Blutgefäß 20 groß ist. Wenn der Flüssigkeitsstrom 17 durch ein Abstellen der Zufuhr in den Schlauchkörper 2 endet und keine Flüssigkeit mehr aus dem Seitenloch 3 austritt, verformt sich das freie Ende 18 elastisch zurück in seine Ausgangsform, d.h., zieht sich zusammen. Darüber hinaus bewegt sich der zuvor verdrängte Bereich des Umlenkkörpers 4 wieder zurück und die gestrafften Bereiche entspannen sich. Die radiale Erstreckung des Umlenkkörpers 4 wird auf diese Weise automatisch vor dem Entfernen reduziert. Wenn der Umlenkkörper 4 dennoch beim Entfernen an einen Rand der Öffnung 21 des Blutgefäßes 20 stößt, kann der Umlenkkörper 4 durch ein Verlagerung in Umfangrichtung und/oder durch ein elastisches Verformung auf eine schonende Art und Weise durch die Öffnung 21 gezogen werden.

Die Figur 2 deutet ebenfalls einen optional implementierbaren Druckmessschlauch 25 an, der zum Seitenloch 3 führt, um einen Flüssigkeitsdruck des Flüssigkeitsstroms 17 messen zu können. Die Querschnittskontur des Schlauchkörpers 2 der Figur 2 kann wie in der Figur 3a gezeigt kreisrund oder bogenförmig sein. Der Schlauchkörper 2 der Figur 2 kann auch wie in der Fig. 3b gezeigt eine dreieckartige Querschnittskontur 31 haben. Der optionale Druckmessschlauch 25 ist auch in den Fig. 3a und 3b angedeutet. Andere Komponenten wie z.B. das Anschlussteil 13, die je nach Blickrichtung zu sehen wären, sind ausgeblendet. Die Wandung 12 des Schlauchkörpers 2 umfasst insbesondere einen integrierten Stützdraht 30, der in den Fig. 3a und 3b ebenfalls nicht illustriert ist. Wenn ein Stützdraht 30 vorgesehen ist, wird dieser ebenso wie der Druckmessschlauch 25 bevorzugt mit einem Elastomer beschichtet oder umspritzt.

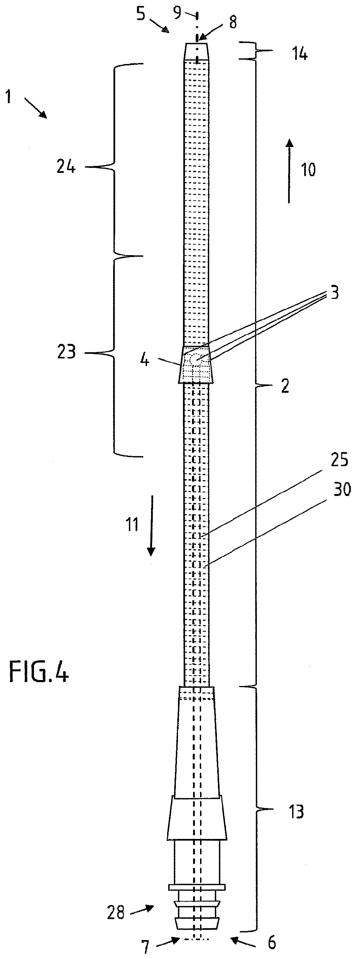

Die Figur 4 zeigt eine medizinische Vorrichtung zur bidirektionalen Perfusion mit sauerstoffangereichertem Blut, insbesondere eine ECMO-Kanüle 1. Der Schlauchkörper 2 erstreckt sich von einem ersten Ende 5 zu einem zweiten Ende 6. Sauerstoffangereichertes Blut kann somit von einem Einlass 7 an dem ersten Ende 5 in eine Vorwärtsrichtung 10 zu einem Auslass 8 an dem zweiten Ende 6 fließen und aus dem Auslass 8 in Vorwärtsrichtung 10 abgegeben werden. Zwischen dem Einlass 7 und dem Auslass 8 sind mindestens drei Seitenlöcher 3 und ein Umlenkkörper 4 vorhanden. Der Umlenkkörper 4 befindet sich außen am Schlauchkörper 2 über allen Seitenlöchern 3, um aus den Seitenlöchern 3 austretendes sauerstoffangereichertes Blut in eine entgegen der Vorwärtsrichtung 10 gerichteten Rückwärtsrichtung 11 umzulenken. Der Umlenkkörper 4 umgibt den Schlauchkörper 2 im Ruhezustand konusförmig, d.h., im einsatzbereiten Zustand ohne äußere mechanisch einwirkende Kräfte. Der Umlenkkörper 4 ist aus einem transparenten Elastomer hergestellt, bevorzugt Polyurethan, so dass die Seitenlöcher 3 von außen durch den Umlenkkörper 4 sichtbar sind. Ein optionaler Stützdraht 30 und ein optionaler Druckmessschlauch 25 können ebenfalls durch eine transparente Elastomer-Schicht sichtbar sein. Der flexible Schlauchkörper 2 erstreckt sich zwischen einem Anschlussteil 13 und einem Kopfteil 14 entlang einer Mittelachse 9. Ein zentraler Bereich 23 erstreckt sich ungefähr über ein Drittel der Längserstreckung des Schlauchkörpers 2. An den zentralen Bereich 23 grenzt ein oberer Bereich 24 bis zum Kopfteil 14 an. Das Anschlussteil 13 umfasst eine bevorzugt sägezahnartige Aufnahme 28 für einen nicht gezeigten Zuleitungsschlauch.

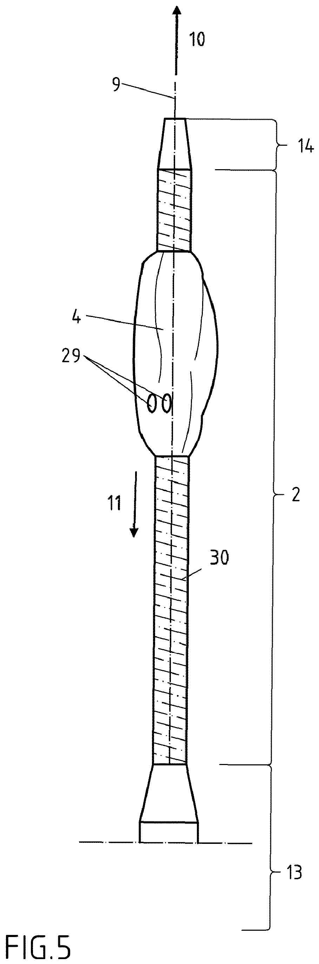

Die Figur 5 zeigt eine medizinische Vorrichtung zur bidirektionalen Perfusion in eine Vorwärtsrichtung 10 und eine Rückwärtsrichtung 11, die einen ballonartigen Umlenkkörper 4 aufweist. Der ballonartige Umlenkkörper 4 ist insbesondere aus einem nicht transparenten Material gefertigt, so dass ein oder mehrere Seitenlöcher 3, die von dem Umlenkkörper 4 überdeckt angeordnet sind - vergleichbar wie in Fig. 2 oder in Fig. 4 gezeigt -, von außen infolge des Überdeckens und/oder Einschließens durch den ballonartigen Umlenkkörper 4 nicht sichtbar sind. Das Material des ballonartigen Umlenkkörpers 4 ist dünn und elastisch. Ein Flüssigkeitsstrom 17 aus mindestens einem Seitenloch 3 - vergleichbar wie in Fig. 2 gezeigt - füllt im Betrieb den ballonartigen Umlenkkörper 4 der Fig. 5 und verlässt den ballonartigen Umlenkkörper 4 durch die Austrittsöffnungen 29. Der ballonartige Umlenkkörper 4 faltet sich dadurch auf und/oder kann je nach Flüssigkeitsdruck elastisch expandiert werden. Durch die rückwärtige Anordnung der Austrittsöffnungen 29 wird der Flüssigkeitsstrom 17 nach hinten in Rückwärtsrichtung 11 umgelenkt. Der flexible Schlauchkörper 2 erstreckt sich zwischen einem Anschlussteil 13 und einem Kopfteil 14 entlang einer Mittelachse 9. Optional kann ein in Fig. 5 angedeuteter Stützdraht 30 zum flexiblen Stabilisieren des Schlauchkörpers 2 vorgesehen sein.

Durch alle in den Figuren gezeigten Ausführungsbeispiele kann eine besonders große Rückflussmenge bei der bidirektionalen Perfusion erzielt werden, ohne ein überhöhtes Risiko einer Gefäßbeschädigung beim Entfernen aus dem Blutgefäß zu erhalten.

Merkmale der Ausführungsbeispiele können einzeln oder in einer Mehrzahl mit den beanspruchten Gegenständen und Aspekten der Erfindung kombiniert werden, sofern nichts Gegenteiliges angegeben wurde. Die beanspruchten Schutzbereiche sind nicht auf die Ausführungsbeispiele beschränkt.

### Bezugszeichenliste:

- 1: ECMO-Kanüle
- 2: Schlauchkörper
- 3: Seitenloch
- 4: Umlenkkörper
- 5: Erstes Ende
- 6: Zweites Ende
- 7: Einlass
- 8: Auslass
- 9: Mittelachse
- 10: Vorwärtsrichtung
- 11: Rückwärtsrichtung
- 12: Wandung des Schlauchkörpers 2
- 13: Anschlussteil
- 14: Kopfteil
- 15: Mit dem Schlauchkörper 2 verbundener Abschnitt
- 16: Von dem Schlauchkörper 2 abstehender Abschnitt
- 17: Aus dem Seitenloch 3 austretender Flüssigkeitsstrom
- 18: Freies Ende des Umlenkkörpers 2
- 19: ECMO-Oxygenator
- 20: Blutgefäß
- 21: Öffnung in Blutgefäß 20
- 22: Patientenhaut
- 23: Zentraler Bereich
- 24: Oberer Bereich
- 25: Druckmessschlauch
- 26: Mantelfläche des Schlauchkörpers 2
- 27: Inzision
- 28: Aufnahme
- 29: Austrittsöffnung
- 30: Stützdraht
- 31: Dreieckartige Querschnittskontur

## Patentansprüche

1. Medizinische Vorrichtung zur bidirektionalen Perfusion mit sauerstoffangereichertem Blut, wobei die Vorrichtung einen Schlauchkörper (2) umfasst und so eingerichtet ist, dass der Schlauchkörper (2) in ein Blutgefäß (20) eingeführt werden kann, wobei sich der Schlauchkörper (2) von einem ersten Ende (5) zu einem zweiten Ende (6) erstreckt, um sauerstoffangereichertes Blut von einem Einlass (7) an dem ersten Ende (5) in eine Vorwärtsrichtung (10) zu einem Auslass (8) an dem zweiten Ende (6) fließen zu lassen und aus dem Auslass (8) in Vorwärtsrichtung (10) abzugeben, wobei zwischen dem Einlass (7) des Schlauchkörpers (2) und dem Auslass (8) des Schlauchkörpers (2) mindestens ein Seitenloch (3) in dem Schlauchkörper (2) mit einem Umlenkkörper (4) vorhanden ist, der sich außen am Schlauchkörper (2) über dem Seitenloch (3) befindet, um aus dem Seitenloch (3) austretendes sauerstoffangereichertes Blut in eine entgegen der Vorwärtsrichtung (10) gerichteten Rückwärtsrichtung (11) umzulenken, wobei die Vorrichtung so konfiguriert ist, dass im Betrieb das sauerstoffangereicherte Blut von dem Einlass (7) sowohl über das mindestens eine Seitenloch (3) in Rückwärtsrichtung (11) als auch aus dem Auslass (8) in Vorwärtsrichtung (10) in das Blutgefäß (20) fließt, wobei das sauerstoffangereicherte Blut hauptsächlich in Vorwärtsrichtung (10) aus dem Auslass (8) in das Blutgefäß (20) fließt, **dadurch gekennzeichnet, dass** der Umlenkkörper (4) den Schlauchkörper (2) in Umfangrichtung umgibt.

2. Medizinische Vorrichtung zur bidirektionalen Perfusion gemäß Anspruch 1, **dadurch gekennzeichnet, dass** der Umlenkkörper (4) konusartig ist.

3. Medizinische Vorrichtung zur bidirektionalen Perfusion gemäß dem vorhergehenden Anspruch, **dadurch gekennzeichnet, dass** der Umlenkkörper (4) trichterartig ist, wobei ein zylindrischer Abschnitt (15) des trichterartigen Umlenkkörpers (4) mit dem Schlauchkörper (2) verbunden ist und ein konischer Abschnitt (16) des trichterartigen Umlenkkörpers (4) von dem Schlauchkörper (2) absteht.

4. Medizinische Vorrichtung zur bidirektionalen Perfusion gemäß einem der vorhergehenden Ansprüche, **dadurch gekennzeichnet, dass** der Umlenkkörper (4) ein am Schlauchkörper (2) befestigter Streifen ist.

5. Medizinische Vorrichtung zur bidirektionalen Perfusion gemäß einem der vorhergehenden Ansprüche, **dadurch gekennzeichnet, dass** Umlenkkörper (4) ballonartig ist.

6. Medizinische Vorrichtung zur bidirektionalen Perfusion gemäß einem der vorhergehenden Ansprüche, **dadurch gekennzeichnet, dass** der Umlenkkörper (4) so eingerichtet ist, dass sich der Umlenkkörper (4) durch eine Druckeinwirkung eines aus dem Seitenloch (3) austretenden Flüssigkeitsstroms (17) bewegen oder verformen lässt.

7. Medizinische Vorrichtung zur bidirektionalen Perfusion gemäß dem vorhergehenden Anspruch, **dadurch gekennzeichnet, dass** die medizinische Vorrichtung so eingerichtet ist, dass je größer eine Durchflussmenge des aus dem Seitenloch (3) austretenden Flüssigkeitsstroms (17) ist, desto größer wird eine rückwärtige Flussquerschnittsfläche, die einen Rückfluss in die Rückwärtsrichtung (11) begrenzt.

8. Medizinische Vorrichtung zur bidirektionalen Perfusion gemäß einem der vorhergehenden Ansprüche, **dadurch gekennzeichnet, dass** der Umlenkkörper (4) eine Dicke von mindestens 0,05 mm und/oder höchstens 0,5 mm hat.

9. Medizinische Vorrichtung zur bidirektionalen Perfusion gemäß einem der vorhergehenden Ansprüche, **dadurch gekennzeichnet, dass** der Umlenkkörper (4) aus einem Material hergestellt ist, das für ein elastisches Verformen im Betrieb vorgesehen ist.

10. Medizinische Vorrichtung zur bidirektionalen Perfusion gemäß einem der vorhergehenden Ansprüche, **dadurch gekennzeichnet, dass** mindestens zwei und/oder höchstens zehn Seitenlöcher (3) von einem einzigen Umlenkkörper (4) überdeckt werden.

11. Medizinische Vorrichtung zur bidirektionalen Perfusion gemäß einem der vorhergehenden Ansprüche, **dadurch gekennzeichnet, dass** mindestens zwei Umlenkkörper (4) vorhanden sind, die in der Vorwärtsrichtung (10) voneinander beabstandet sind und sich jeweils über mindestens einem Seitenloch (3) befinden.

12. Medizinische Vorrichtung zur bidirektionalen Perfusion gemäß einem der vorhergehenden Ansprüche, **dadurch gekennzeichnet, dass** eine Vielzahl von Stellen in einer Wandung (12) des Schlauchkörpers (2) zum Einbringen eines Seitenloches (3) vorgesehen ist und nur eine oder mehrere Stellen davon zum Erhalt eines Seitenloches (3) gelocht sind.

13. Medizinische Vorrichtung zur bidirektionalen Perfusion gemäß einem der vorhergehenden Ansprüche, **dadurch gekennzeichnet, dass** ein Druckmessschlauch (25) vorgesehen ist, der in Vorwärtsrichtung (10) zum Seitenloch (3) führt, damit ein Flüssigkeitsdruck des aus dem Seitenloch (3) austretenden Flüssigkeitsstroms (17) gemessen werden kann.

14. Medizinische Vorrichtung zur bidirektionalen Perfusion gemäß einem der vorhergehenden Ansprüche, **dadurch gekennzeichnet, dass** der Schlauchkörper (3) eine dreieckartige Querschnittskontur (31) der äußeren Mantelfläche (26) aufweist.

15. Perfusionssystem mit der medizinischen Vorrichtung zur bidirektionalen Perfusion gemäß einem der vorhergehenden Ansprüche und einem Oxygenator, der an den Einlass (7) der medizinischen Vorrichtung zur bidirektionalen Perfusion angeschlossen ist, so dass sauerstoffangereichertes Blut in den Einlass (7) gepumpt werden kann.

## Claims

1. Medical device for bidirectional perfusion with oxygen-enriched blood, the device comprising a tubular body (2) and being configured such that the tubular body (2) can be inserted into a blood vessel (20), wherein the tubular body (2) extends from a first end (5) to a second end (6) to allow oxygen-enriched blood to flow from an inlet (7) at the first end (5) in a forward direction (10) to an outlet (8) at the second end (6) and to be dispensed from the outlet (8) in the forward direction (10), wherein between the inlet (7) of the tubular body (2) and the outlet (8) of the tubular body (2) there is at least one side hole (3) in the tubular body (2) with a deflecting body (4) which is located on the outside of the tubular body (2) above the side hole (3), in order to deflect oxygen-enriched blood emerging from the side hole (3) in a backward direction (11) directed opposite to the forward direction (10), wherein the device is configured such that in operation the oxygen-enriched blood flows from the inlet (7) both via the at least one side hole (3) in the backward direction (11) and from the outlet (8) in the forward direction (10) into the blood vessel (20), wherein the oxygen-enriched blood flows mainly in the forward direction (10) from the outlet (8) into the blood vessel (20), **characterized in that** the deflecting body (4) surrounds the tubular body (2) in the circumferential direction.

2. Medical device for bidirectional perfusion according to claim 1, **characterized in that** the deflecting body (4) is cone-like.

3. Medical device for bidirectional perfusion according to the preceding claim, **characterized in that** said deflecting body (4) is funnel-shaped, wherein a cylindrical portion (15) of said funnel-shaped deflecting body (4) is connected to said tubular body (2) and a conical portion (16) of said funnel-shaped deflecting body (4) protrudes from said tubular body (2).

4. Medical device for bidirectional perfusion according to one of the preceding claims, **characterized in that** the deflecting body (4) is a strip attached to the tubular body (2).

5. Medical device for bidirectional perfusion according to one of the preceding claims, **characterized in that** deflecting body (4) is balloon-like.

6. Medical device for bidirectional perfusion according to one of the preceding claims, **characterized in that** the deflecting body (4) is configured such that the deflecting body (4) can be moved or deformed by a pressure action of a fluid flow (17) emerging from the side hole (3).

7. Medical device for bidirectional perfusion according to the preceding claim, **characterized in that** the medical device is configured such that the larger a flow rate of the fluid flow (17) emerging from the side hole (3), the larger a backward cross-sectional flow area limiting a back flow in the backward direction (11) becomes.

8. Medical device for bidirectional perfusion according to one of the preceding claims, **characterized in that** the deflecting body (4) has a thickness of at least 0.05 mm and/or at most 0.5 mm.

9. Medical device for bidirectional perfusion according to one of the preceding claims, **characterized in that** the deflecting body (4) is produced from a material which is provided for elastic deformation in operation.

10. Medical device for bidirectional perfusion according to one of the preceding claims, **characterized in that** at least two and/or at most ten side holes (3) are covered by a single deflecting body (4).

11. Medical device for bidirectional perfusion according to one of the preceding claims, **characterized in that** at least two deflecting bodies (4) are present, which are spaced apart from each other in the forward direction (10) and are each located above at least one side hole (3).

12. Medical device for bidirectional perfusion according to one of the preceding claims, **characterized in that** a plurality of sites are provided in a wall (12) of the tubular body (2) for insertion of a side hole (3) and only one or more sites thereof are perforated to obtain a side hole (3).

13. Medical device for bidirectional perfusion according to one one of the preceding claims, **characterized in that** a pressure measuring tube (25) is provided leading in a forward direction (10) to the side hole (3) such that a fluid pressure of the fluid flow (17) emerging from the side hole (3) can be measured.

14. Medical device for bidirectional perfusion according to one of the preceding claims, **characterized in that** the tubular body (3) has a triangle-like cross-sectional contour (31) of the outer lateral surface (26).

15. Perfusion system comprising the medical device for bidirectional perfusion according to one of the preceding claims and an oxygenator connected to the inlet (7) of the medical device for bidirectional perfusion so that oxygen-enriched blood can be pumped into the inlet (7).

## Revendications

1. Dispositif médical pour la perfusion bidirectionnelle de sang enrichi en oxygène, dans lequel le dispositif comprend un corps de tuyau (2) et est adapté de sorte que le corps de tuyau (2) peut être inséré dans un vaisseau sanguin (20), dans lequel le corps de tuyau (2) s'étend d'une première extrémité (5) à une seconde extrémité (6), pour faire le sang enrichi en oxygène s'écouler d'une entrée (7) à la première extrémité (5) dans une direction vers l'avant (10) vers une sortie (8) à la deuxième extrémité (6) et sortir de la sortie (8) dans la direction vers l'avant (10), dans lequel, entre l'entrée (7) du corps de tuyau (2) et la sortie (8) du corps de tuyau (2), il existe au moins un trou latéral (3) dans le corps de tuyau (2) avec un corps de déviation (4), qui se trouve à l'extérieur au corps de tuyau (2) au-dessus du trou latéral (3), pour dévier le sang enrichi en oxygène sortant du trou latéral (3) dans une direction vers l'arrière (11) dirigée à l'opposé de la direction avant (10), dans lequel le dispositif est configuré de telle sorte qu'en fonctionnement le sang enrichi en oxygène s'écoule depuis l'entrée (7) à la fois via le au moins un trou latéral (3) dans la direction vers l'arrière (11) et depuis la sortie (8) dans la direction vers l'avant (10) dans le vaisseau sanguin (20), dans lequel le sang enrichi en oxygène s'écoulant principalement dans la direction vers l'avant (10) depuis la sortie (8) dans le vaisseau sanguin (20), **caractérisé en ce que** le corps de déviation (4) entoure le corps de tuyau (2) dans la direction circonférentielle.

2. Dispositif médical de perfusion bidirectionnelle selon la revendication 1, **caractérisé en ce que** le corps de déviation (4) est de type conique.

3. Dispositif médical de perfusion bidirectionnelle selon la revendication précédente, **caractérisé en ce que** le corps de déviation (4) est en forme d'entonnoir, dans lequel une partie cylindrique (15) du corps de déviation (4) en forme d'entonnoir est reliée au corps de tuyau (2) et une partie conique (16) du corps de déviation (4) en forme d'entonnoir se dresse du corps de tuyau (2).

4. Dispositif médical de perfusion bidirectionnelle selon l'une des revendications précédentes, **caractérisé en ce que** le corps de déviation (4) est une bande fixée au corps de tuyau (2).

5. Dispositif médical de perfusion bidirectionnelle selon l'une des revendications précédentes, **caractérisé en ce que** le corps de déviation (4) est en forme de ballon.

6. Dispositif médical de perfusion bidirectionnelle selon l'une des revendications précédentes, **caractérisé en ce que** le corps de déviation (4) est agencé de manière à ce que le corps de déviation (4) peut être déplacé ou déformé par un effet de pression d'un flux de liquide (17) sortant du trou latéral (3).

7. Dispositif médical de perfusion bidirectionnelle selon la revendication précédente, **caractérisé en ce que** le dispositif médical est agencé de telle sorte que plus un débit du flux de liquide (17) sortant du trou latéral (3) est important, plus une surface de section transversale d'écoulement arrière limitant un reflux dans la direction vers l'arrière (11) est importante.

8. Dispositif médical de perfusion bidirectionnelle selon l'une des revendications précédentes, **caractérisé en ce que** le corps de déviation (4) a une épaisseur d'au moins 0,05 mm et/ou d'au plus 0,5 mm.

9. Dispositif médical de perfusion bidirectionnelle selon l'une des revendications précédentes, **caractérisé en ce que** le corps de déviation (4) est réalisé dans un matériau prévu pour la déformation élastique en fonctionnement.

10. Dispositif médical de perfusion bidirectionnelle selon l'une des revendications précédentes, **caractérisé en ce qu'**au moins deux et/ou au plus dix orifices latéraux (3) sont recouverts par un seul corps de déviation (4).

11. Dispositif médical de perfusion bidirectionnelle selon l'une des revendications précédentes, **caractérisé en ce qu'**il existe au moins deux corps de déviation (4) qui sont espacés l'un de l'autre dans la direction avant (10) et qui se trouvent chacun au-dessus d'au moins un trou latéral (3).

12. Dispositif médical de perfusion bidirectionnelle selon l'une des revendications précédentes, **caractérisé en ce qu'**une pluralité d'emplacements sont prévus dans une paroi (12) du corps de tuyau (2) pour l'insertion d'un trou latéral (3) et **en ce qu'**un ou plusieurs emplacements seulement de ceux-ci sont perforés pour obtenir un trou latéral (3).

13. Dispositif médical de perfusion bidirectionnelle selon l'une quelconque des revendications précédentes, **caractérisé en ce qu'**il est prévu un tuyau de mesure de pression (25) qui mène au trou latéral (3) dans la direction vers l'avant (10) afin de pouvoir mesurer une pression de liquide du flux de liquide (17) sortant du trou latéral (3).

14. Dispositif médical de perfusion bidirectionnelle selon l'une quelconque des revendications précédentes, **caractérisé en ce que** le corps de tuyau (3) présente un contour de section transversale (31) de type triangulaire de la surface d'enveloppe externe (26).

15. Système de perfusion comprenant le dispositif médical de perfusion bidirectionnelle selon l'une des revendications précédentes et un oxygénateur qui est raccordé à l'entrée (7) du dispositif médical de perfusion bidirectionnelle, de sorte que du sang enrichi en oxygène peut être pompé dans l'entrée (7).
